Europäisches Patentamt

European Patent Office

Office européen des brevets

(19)

(11) Publication number : **0 658 568 A1**

(12) **EUROPEAN PATENT APPLICATION**

(21) Application number : **94308950.8**

(22) Date of filing : **02.12.94**

(51) Int. Cl.⁶ : **C07K 14/605, A61K 47/48, A61K 47/02**

(30) Priority : **09.12.93 US 164277**

(43) Date of publication of application :
**21.06.95 Bulletin 95/25**

(84) Designated Contracting States :
**AT BE CH DE DK ES FR GB GR IE IT LI LU NL PT SE**

(71) Applicant : **ELI LILLY AND COMPANY**
**Lilly Corporate Center**
**Indianapolis Indiana 46285 (US)**

(72) Inventor : **Galloway, John Allison**
**215 Olde Mill Cove**
**Indianapolis, Indiana 46260 (US)**
Inventor : **Hoffmann, James Arthur**
**4272 Woodland Streams Drive**
**Greenwood, Indiana 46143 (US)**

(74) Representative : **Tapping, Kenneth George et al**
**Lilly Industries Limited**
**European Patent Operations**
**Erl Wood Manor**
**Windlesham Surrey GU20 6PH (GB)**

(54) **Glucagon-like insulinotropic peptides, compositions and methods.**

(57)   The present invention provides novel compounds comprising certain GLP-1 molecules complexed with a divalent metal cation, pharmaceutical compositions thereof, and methods of using such compounds for enhancing the expression of insulin in B-type islet cells and for treating maturity onset diabetes mellitus in mammals, particularly humans.

EP 0 658 568 A1

This invention relates to the field of pharmaceutical and organic chemistry and provides novel compounds, and pharmaceutical compositions thereof, which are useful for enhancing the expression of insulin from mammalian pancreatic B-type islet cells and for treating maturity onset diabetes mellitus in a mammal.

The endocrine secretions of the pancreatic islets are under complex control not only by blood-borne metabolites (glucose, amino acids, catecholamines, etc.), but also by local paracrine influences. The major pancreatic islet hormones (glucagon, insulin and somatostatin) interact amongst their specific cell types (A, B, and D cells, respectively) to modulate secretory responses mediated by the aforementioned metabolites. Although insulin secretion is predominantly controlled by blood levels of glucose, somatostatin inhibits glucose-mediated insulin secretory responses. In addition to the proposed interislet paracrine regulation of insulin secretion, there is evidence to support the existence of insulinotropic factors in the intestine. This concept originates from the observations that glucose taken orally is a much more potent stimulant of insulin secretion than is a comparable amount of glucose given intravenously.

The human hormone glucagon is a 29-amino acid peptide hormone produced in the A-cells of the pancreas. The hormone belongs to a multi-gene family of structurally related peptides that include secretin, gastric inhibitory peptide, vasoactive intestinal peptide and glicentin. These peptides variously regulate carbohydrate metabolism, gastrointestinal mobility and secretory processing. The principal recognized actions of pancreatic glucagon, however, are to promote hepatic glycogenolysis and glyconeogenesis, resulting in an elevation of blood sugar levels. In this regard, the actions of glucagon are counter regulatory to those of insulin and may contribute to the hyperglycemia that accompanies Diabetes mellitus [(Lund, P.K., et al., Proc. Natl. Acad. Sci. U.S.A., 79:345-349 (1982)].

Glucagon has been found to be capable of binding to specific receptors which lie on the surface of insulin producing cells. Glucagon, when bound to these receptors, stimulates the rapid synthesis of cAMP by these cells. cAMP, in turn, has been found to stimulate insulin expression [Korman, L.Y., et al., Diabetes, 34:717-722 (1985)]. Insulin acts to inhibit glucagon synthesis [Ganong, W.F., Review of Medical Physiology, Lange Publications, Los Altos, California, p. 273(1979)]. Thus, the expression of glucagon is carefully regulated by insulin, and ultimately by the serum glucose level.

The glucagon gene is initially translated from a 360 base pair precursor to form the polypeptide, preproglucagon [Lund, et al., Proc. Natl. Acad. Sci. U.S.A. 79:345-349 (1982)]. This polypeptide is subsequently processed to form proglucagon. Patzelt, C., et al., Nature, 282:260-266 (1979), demonstrated that proglucagon was subsequently cleaved into glucagon and a second polypeptide. Subsequent work by Lund, P.K., et al., Lopez L.C., et al., Proc. Natl. Acad. Sci. U.S.A., 80:5485-5489 (1983), and Bell, G.I., et al., Nature 302:716-718 (1983), demonstrated that the proglucagon molecule was cleaved immediately after lysine-arginine dipeptide residues. Studies of proglucagon produced by channel catfish (Ictalurus punctata) indicated that glucagon from this animal was also proteolytically cleaved after adjacent lysine-arginine dipeptide residues [Andrews P. C., et al., J. Biol. Chem., 260:3910-3914 (1985), Lopez, L.C., et al., Proc. Natl. Acad. Sci. U.S.A., 80:5485-5489 (1983)]. Bell, G.I., et al., supra, discovered that mammalian proglucagon was cleaved at lysine-arginine or arginine-arginine dipeptides, and demonstrated that the proglucagon molecule contained three discrete and highly homologous peptide molecules which were designated glucagon, glucagon-like peptide 1 (GLP-1) and glucagon-like peptide 2 (GLP-2). Lopez, et al., concluded that glucagon-like peptide 1 was 37 amino acid residues long and that glucagon-like peptide 2 was 34 amino acid residues long. Analogous studies on the structure of rat preproglucagon revealed a similar pattern of proteolytic cleavage between adjacent lysine-arginine or arginine-arginine dipeptide residues, resulting in the formation of glucagon, GLP-1 and GLP-2 [Heinrich, G., et al., Endocrinol., 115:2176-2181 (1984)]. Human, rat, bovine, and hamster sequences of GLP-1 have been found to be identical [Ghiglione, M., et al., Diabetologia, 27:599-600 (1984)].

The conclusion reached by Lopez, et al., regarding the size of GLP-1 was confirmed by the work of Uttenthal, L.O., et al., J. Clin. Endocrinol. Metabol., 61:472-479 (1985).

Uttenthal, et al., examined the molecular forms of GLP-1 which were present in the human pancreas. Their research shows that GLP-1 and GLP-2 are present in the pancreas as 37 amino acid and 34 amino acid peptides, respectively.

The similarity between GLP-1 and glucagon suggested to early investigators that GLP-1 might have biological activity. Although some investigators found that GLP-1 could induce rat brain cells to synthesize cAMP [Hoosein, N.M., et al., Febs Lett. 178:83-86 (1984)], other investigators failed to identify any physiological role for GLP-1 (Lopez, L.C., et al.). The failure to identify any physiological role for GLP-1 caused some investigators to question whether GLP-1 was in fact a hormone and whether the relatedness between glucagon and GLP-1 might be artifactual.

Variants of GLP-1 (7-37) and analogs thereof, also have been disclosed. These variants and analogs include, for example, $Gln^9$-GLP-1 (7-37), $D$-$Gln^9$-GLP-1 (7-37), acetyl-$Lys^9$-GLP-1 (7-37), $Thr^{16}$-$Lys^{18}$-GLP-1 (7-37), $Lys^{18}$-GLP-1 (7-37) and the like, and derivatives thereof including, for example, acid addition salts, carboxylate salts, lower alkyl esters, and amides [see, eg., WO 91/11457]. Generally, the various disclosed forms of GLP-1 are known to stimulate insulin secretion (insulinotropic action) and cAMP formation [see, e g., Mojsov, S., *Int. J. Peptide Protein Research*, 40:333-343 (1992)].

More importantly, multiple authors have demonstrated the nexus between laboratory experimentation and mammalian, particularly human, insulinotropic responses to exogenous administration of GLP-1, particularly GLP-1 (7-36)amide and GLP-1 (7-37) [see, e.g., Nauck, MA., *et al.*, *Diabetologia*, 36:741-744 (1993); Gutniak, M., *et al.*, *New England J. of Medicine*, 326(20):1316-1322 (1992); Nauck, M.A., *et al.*, *J. Clin. Invest.*, 91:301-307 (1993); and Thorens, B., *et al.*, *Diabetes*, 42:1219-1225(1993)].

More particularly, the fundamental defects identified as causing hyperglycemia in maturity onset diabetes are impaired secretion of endogenous insulin and resistance to the effects of insulin by muscle and liver [Galloway, J.S., *Diabetes Care*, 13:1209-1239, (1990)]. The latter defect results in excessive production of glucose from the liver. Thus, whereas a normal individual releases glucose at the rate of approximately 2 mg/kg/minute, in patients with maturity onset diabetes, this amount usually exceeds 2.5 mg/kg/minute resulting in a net excess of at least 70 grams of glucose per 24 hours. The fact that there exists exceedingly high correlations between hepatic glucose production, the fasting blood glucose and overall metabolic control as indicated by glycohemoglobin measurements [Galloway, J.A., supra; and Galloway, *J.A., et al.*, *Clin. Therap.*, 12:460-472 (1990)], it is readily apparent that control of the fasting blood glucose is a *sine quo* non for achieving overall normalization of metabolism sufficient to prevent the complication of hyperglycemia. In view of the fact that present forms of insulin rarely normalize hepatic glucose production without producing significant hyperinsulinemia and hypoglycemia (Galloway, J.A., and Galloway, J.A., *et al.*, supra) alternative approaches are needed.

Intravenous infusions of GLIP-1 (7-36)amide to produce twice normal serum concentrations have been demonstrated to produce the effects indicated in the table below:

| | Normal Subjects | Patients With Maturity Onset Diabetes |
|---|---|---|
| Meal glycemia (1) | Unchanged | Reduced |
| Fasting glycemia (2) | -- | Reduced |
| Fasting glucagon (2) | -- | Reduced |
| Post-prandial glucagon (1) | -- | Reduced |
| Endogenous insulin secretion in response to a meal (1) | Unchanged | Increased |
| Free fatty acids | Reduced (3) | Reduced (2) |

(1) Gutniak, M., *et al.*, supra.

(2) Nauck, M.A., *et al.*, *Diabetologia*, supra.

(3) Orskov, C., *et al.*, *Diabetes*, 42:658-661, (1993).

However, the long-term stability of GLP-1, particularly GLP-1 as a component of a pharmaceutical composition for administration to mammals, is questionable. In fact, when stored at the low temperature of 4°C, by-products of GLP-1 (7-37) have been found as early as eleven months after sample preparation (Mojsov, S., supra). Thus, there exists a need for a more stable GLP-1 compound which can safely be administered to mammals in need of such treatment.

Furthermore, the biological half-life of GLP-1 molecules, particularly those molecules which are affected by the activity of dipeptidyl-peptidase IV (DPP IV) is quite short. For example, the biological half-life of GLP-1 (7-37) is a mere 3 to 5 minutes (U.S. Pat. No. 5,118,666), and is further influenced by its rapid absorption following parenteral administration to a mammal. Thus, there also exists a need for a GLP-1 compound or formulation which delays absorption following such administration.

Accordingly, the present invention provides compounds and formulations which satisfy the aforementioned stability requirements. The compounds of the present invention also provide delayed absorption following parenteral administration and, consequently, should have extended biological half-lives. Also provided are pharmaceutical compositions of the compounds of the present invention, as well as methods for using such compounds.

The present invention provides a compound comprising a GLP-1 molecule having at least one histidine functionality or a modified histidine functionality and further having an isoelectric point in the range from about 6.0 to about 9.0, complexed with a divalent metal cation.

Also provided by the present invention is a pharmaceutical composition comprising a compound of the present invention in combination with a pharmaceutically acceptable carrier, diluent, or excipient.

The present invention further provides a method for enhancing the expression of insulin comprising providing to a mammalian pancreatic B-type islet cell an effective amount of a compound of the present invention, as well as a method for treating maturity onset diabetes mellitus in a mammal in need of such treatment comprising administering an effective amount of a compound of the present invention to said mammal.

One aspect of the present invention provides a compound comprising a GLP-1 molecule having at least one histidine functionality or a modified histidine functionality and further having an isoelectric point in the range from about 6.0 to about 9.0, complexed with a divalent metal cation.

As used in the present specification, the term "GLP-1 molecule" refers to naturally-occurring GLP-1 (7-36)amide, GLP-1 (7-37), natural and unnatural functional analogs and derivatives thereof, and salts thereof. The amino acid sequence of GLP-1 (7-36)amide is well known in the art, but is presented below as a convenience to the reader:

$$His^7\text{-}Ala\text{-}Glu\text{-}Gly^{10}\text{-}Thr\text{-}Phe\text{-}Thr\text{-}Ser\text{-}Asp^{15}\text{-}Val\text{-}Ser\text{-}Ser\text{-}Tyr\text{-}$$
$$Leu^{20}\text{-}Glu\text{-}Gly\text{-}Gln\text{-}Ala\text{-}Ala^{25}\text{-}Lys\text{-}Glu\text{-}Phe\text{-}Ile\text{-}Ala^{30}\text{-}Trp\text{-}$$
$$Leu\text{-}Val\text{-}Lys\text{-}Gly^{35}\text{-}Arg\text{-}NH_2.$$

For GLP-1 (7-37), the carboxy-terminal amide functionality of $Arg^{36}$ is displaced with Gly at the 37th position of the GLP-1 (7-36)amide molecule.

In addition, the existence and preparation of a multitude of protected, unprotected, and partially protected natural and unnatural functional analogs and derivatives of GLP-1 (7-36)amide and GLP-1 (7-37) molecules have been described in the art [see, e.g., U.S. Pat. No. 5,120,712 and 5,118,666, which are herein incorporated by reference, and Orskov, C., et al., J. Biol. Chem., 264(22):12826-12829 (1989) and WO 91/11457 (Buckley, D.I., et al., published August 8, 1991)].

As known in the art, amino acid residues may be in their protected form in which both amino and carboxy groups possess appropriate protecting groups, partially-protected form in which either amino or carboxy groups possess appropriate protecting groups, or unprotected form in which neither amino nor carboxy groups possess an appropriate protecting group. Numerous reactions for the formation and removal of such protecting groups are described in a number of standard works including, for example, "Protective Groups in Organic Chemistry", Plenum Press (London and New York, 1973); Green, T.H., "Protective Groups in Organic Synthesis", Wiley (New York, 1981); and "The Peptides", Vol. I, Schröder and Lübke, Academic Press (London and New York, 1965).

Representative amino protecting groups include, for example, formyl, acetyl, isopropyl, butoxycarbonyl, fluorenylmethoxycarbonyl, carbobenzyloxy, and the like.

Representative carboxy protecting groups include, for example, benzyl ester, methyl ester, ethyl ester, t-butyl ester, p-nitro phenyl ester, and the like.

In addition to protected forms in which both amino and carboxy groups possess appropriate protecting groups, the term "protected" also refers to those GLP-1 molecules in which the activity of dipeptidyl-peptidase IV is resisted or inhibited [see, e.g., Mentlein, R., et al., Eur. J. Biochem., 214:829-835 (1993)]. In addition to GLP-1 (7-36)amide and glycine-8-($Gly^8$)-GLP-1 (7-36)amide, GLP-1 molecules which are protected from the activity of DPP IV are preferred.

Derivatives of naturally-occurring GLP-1 molecules are those peptides which are obtained by fragmenting a naturally-occurring sequence, or are synthesized based upon a knowledge of the sequence of the naturally-occurring amino acid sequence of the genetic material (DNA or RNA) which encodes this sequence. The term "derivatives" also includes chemical modification of natural or unnatural GLP-1 molecules. Processes for preparing these derivatives are well known to organic and peptide chemists of ordinary skill (see, e.g., WO 91/11457, supra).

GLP-1 molecules of the present invention also include analogs of GLP-1 (7-36)amide and GLP-1 (7-37) in which one or more amino acids which are not present in the original sequence are added or deleted, and derivatives thereof.

Furthermore, the present invention includes a salt form of a GLP-1 molecule. A GLP-1 of this invention can possess a sufficiently acidic, a sufficiently basic, or both functional groups, and accordingly react with any of a number of inorganic bases, and inorganic and organic acids, to form a salt. Acids commonly employed to form acid addition salts are inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, p-bromophenyl-sulfonic acid, carbonic acid, succinic acid, citric acid, benzoic acid, acetic acid, and the like. Examples of such salts include the sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gamma-hydroxybutyrate, glycolate, tartrate, methanesulfonate, propanesulfonate, naphthalene-1-sulfonate, naphthalene-2-sulfonate, mandelate, and the like. Preferred acid addition salts are those formed with mineral acids such as hydrochloric acid and hydrobromic acid, and, especially, hydrochloric acid.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, and the like. The salt forms are particularly preferred.

Of course, when the compounds of this invention are used for pharmacotherapeutic purposes, those compounds may also be in the form of a salt, but the salt must be pharmaceutically acceptable.

Thus, GLP-1 molecules of the present invention include *inter alia*, those GLP-1 molecules which functionally demonstrate insulinotropic activity. The term "insulinotropic activity" relates to the ability of a substance to stimulate, or cause the stimulation of, the synthesis or expression of the hormone insulin.

The insulinotropic property of a compound may be determined by providing that compound to animal cells, or injecting that compound into animals and monitoring the release of immunoreactive insulin (IRI) into the media or circulatory system of the animal, respectively. The presence of IRI is detected through the use of a radioimmunoassay which can specifically detect insulin.

Although any radioimmunoassay capable of detecting the presence of IRI may be employed, it is preferable to use a modification of the assay method of Albano, J.D.M., *et al.*, *Acta Endocrinol.*, 70:487-509 (1972). In this modification, a phosphate/albumin buffer with a pH of 7.4 is employed. The incubation is prepared with the consecutive addition of 500 μl of phosphate buffer, 50 μl of perfusate sample or rat insulin standard in perfusate, 100 μl of anti-insulin antiserum (Wellcome Laboratories; 1:40,000 dilution), and 100 μl of [125I] insulin, giving a total volume of 750 μl in a 10x75 mm disposable glass tube. After incubation for 2-3 days at 4° C, free insulin is separated from antibody-bound insulin by charcoal separation. The assay sensitivity is 1-2 uU/mL. In order to measure the release of IRI into the cell culture medium of cells grown in tissue culture, one preferably incorporates radioactive label into proinsulin. Although any radioactive label capable of labelling a polypeptide can be used, it is preferable to use $^3$H leucine in order to obtain labelled proinsulin. Labelling can be done for any period of time sufficient to permit the formation of a detectably labelled pool of proinsulin molecules; however, it is preferable to incubate cells in the presence of radioactive label for a 60 minute time period.

Although many cell lines capable of expressing insulin can be used for determining whether a compound has an insulinotropic effect, it is preferable to use rat insulinoma cells, and especially RIN-38 rat insulinoma cells. Such cells can be grown in any suitable medium; however, it is preferable to use DME medium containing 0.1% BSA and 25 mM glucose.

The insulinotropic property of a compound may also be determined by pancreatic infusion. The *in situ* isolated perfused rat pancreas preparation is a modification of the method of Penhos, J.C., *et al.*, *Diabetes*, 18:733-738 (1969). Fasted male Charles River strain albino rats, weighing 350-600 g, are anesthetized with an intraperitoneal injection of Amytal Sodium (Eli Lilly and Co.: 160 ng/kg). Renal, adrenal, gastric, and lower colonic blood vessels are ligated. The entire intestine is resected except for about four cm of duodenum and the descending colon and rectum. Therefore, only a small part of the intestine is perfused, minimizing possible interference by enteric substances with glucagon-like immunoreactivity. The perfusate is a modified Krebs-Ringer bicarbonate buffer with 4% dextran T70 and 0.2% bovine serum albumin (fraction V), and is bubbled with 95% $O_2$ and 5% $CO_2$. A nonpulsatile flow, 4-channel roller bearing pump (Buchler polystatic, Buchler Instruments Division, Nuclear-Chicago Corp.) is used, and a switch from one perfusate source to another is accomplished by switching a 3-way stopcock. The manner in which perfusion is performed, monitored, and analyzed follow the method of Weir, G.C., *et al.*, *J. Clin. Inestigat.* 54:1403-1412 (1974), which is hereby incorporated by reference.

The GLP-1 molecules of the present invention are required to possess at least one histidine functionality, preferably at the 7-position as shown above. However, the histidine functionality may be located at any position within a GLP-1 molecule.

Alternatively, GLP-1 molecules of the present invention may possess at least one modified histidine functionality in lieu of the required histidine functionality, and may be located at any position within a GLP-1 molecule. Preferably, a modified histidine functionality is located at the 7-position of a GLP-1 molecule.

The term "modified histidine" refers to a histidine functionality which has been chemically or biologically altered or an altered histidine functionality which has been synthesized de novo, but which retains its metal binding properties.

Numerous such modified histidine functionalities and their preparation are known in the art and include, for example, D-histidine (WO 91/11457), desamino histidine (WO 92/18531), 2-amino-histidine [Levine-Pinto, H., et al., Biochem. Biophys. Res. Commun., 103(4):1121-1130 (1981)], β-hydroxy-L-histidine [Owa, T, et al., Chemistry Letters, pp. 1873-1874(1988)], L-homohistidine [Altman, J., et al., Synthetic Commun., 19(11&12):2069-2076 (1989)], α-fluoromethyl-histidine (U.S. Pat. NO. 4,347,374), and α-methylhistidine [O'Donnell, M.J., Synthetic Commun., 19(7&8):1157-1165 (1989)].

The GLP-1 molecules of the present invention further are required to have an isoelectric point in the range from about 6.0 to about 9.0. Numerous GLP-1 molecules having an isoelectric point in this range have been disclosed and include, for example:

GLP-1 (7-36)amide
$Gly^8$-GLP-1 (7-36)amide
$Gln^9$-GLP-1 (7-37)
D-$Gln^9$-GLP-1 (7-37)
acetyl-$Lys^9$-GLP-1 (7-37)
$Thr^9$-GLP-1 (7-37)
D-$Thr^9$-GLP-1 (7-37)
$Asn^9$-GLP-1 (7-37)
D-$Asn^9$-GLP-$^1$ (7-37)
$Ser^{22}$-$Arg^{23}$-$Arg^{24}$-$Gln^{26}$-GLP-1 (7-37)
$Thr^{16}$-$Lys^{18}$-GLP-1 (7-37)
$Lys^{18}$-GLP-1 (7-37)
$Arg^{23}$-GLP-1 (7-37)
$Arg^{24}$-GLP-1 (7-37),

and the like (see, e.g., WO 91/11457, supra). In addition, GLP-1 molecules of the present invention, when possessing each of the above-referenced modified histidine functionalities in lieu of the histidine functionality, have isoelectric points which fall within the above-defined range. Methods for calculating or experimentally determining the isoelectric point of other GLP-1 molecules are known to one of ordinary skill in the art.

Methods for preparing the GLP-1 molecules of the present invention also are well known to an ordinarily skilled peptide chemist.

In one method, GLP-1 molecules are prepared by the well-known solid phase peptide synthetic schemes described by Merrifield, J.M., Chem. Soc., 85:2149 (1962), and Stewart and Young, Solid Phase Peptide Synthesis, pp. 24-66, Freeman (San Francisco, 1969). However, it also is possible to obtain fragments of the proglucagon polypeptide or of GLP-1 (1-37) by fragmenting the naturally-occurring amino acid sequence using, for example, a proteolytic enzyme. Further, it is possible to obtain the desired fragments of the proglucagon peptide or of GLP-1 (1-37) through the use of recombinant DNA technology as disclosed by Maniatis, T., et al., Molecular Biology: A Laboratory Manual, CSH (Cold Spring Harbor, 1982).

Once the desired GLP-1 molecule is prepared, providing it possesses at least one histidine functionality or modified histidine functionality and further has an isoelectric point in the range from about 6.0 to about 9.0, compounds of the present invention are prepared by complexing a desired GLP-1 molecule with a divalent metal cation via well known methods in the art. Such metal cations include, for example, $Zn^{++}$, $Mn^{++}$, $Fe^{++}$, $Co^{++}$, $Cd^{++}$, $Ni^{++}$, and the like. Of the metal cations, $Zn^{++}$ is preferred.

Generally, a desired GLP-1 molecule, having the required histidine or modified histidine functionality and isoelectric point, is combined with a mixture of an appropriate buffer and an appropriate form of a metal cation.

Appropriate buffers are those which will maintain the mixture at a pH range from about 6.0 to about 9.0, but which will not interfere with the reaction. Preferred buffers include Goode's buffers, particularly HEPES, and Tris and Tris acetate.

Appropriate forms of metal cations are any form of a divalent metal cation which is available to form a complex with a GLP-1 molecule of the present invention. Preferably, a divalent metal cationic salt such as zinc chloride is provided in excess to provide a molar ratio of up to about 50 molecules of a divalent metal cation for each molecule of GLP-1 substrate.

The temperature employed in this step is that which is sufficient to effect completion of the reaction. Typically, the reaction is run at ambient temperature.

The product of the present reaction, a crystalline or amorphous suspension, is isolated and purified using standard techniques.

The present invention also provides pharmaceutical compositions comprising a compound of the present invention in combination with a pharmaceutically acceptable carrier, diluent, or excipient. Such pharmaceutical compositions are prepared in a manner well known in the pharmaceutical art, and are administered individually or in combination with other therapeutic agents, preferably via parenteral routes. Especially preferred routes include intramuscular and subcutaneous administration.

Parenteral daily dosages, preferably a single, daily dose, are in the range from about 1 pg/kg to about 1,000 μg/kg of body weight, although lower or higher dosages may be administered. The required dosage will depend upon the severity of the condition of the patient and upon such criteria as the patient's height, weight, sex, age, and medical history.

In making the compositions of the present invention, the active ingredient, which comprises at least one compound of the present invention, is usually mixed with an excipient or diluted by an excipient. When an excipient is used as a diluent, it may be a solid, semi-solid, or liquid material which acts as a vehicle, carrier, or medium for the active ingredient.

In preparing a formulation, it may be necessary to mill the active compound to provide the appropriate particle size prior to combining with the other ingredients. If the active compound is substantially insoluble, it ordinarily is milled to particle size of less than about 200 mesh. If the active compound is substantially water soluble, the particle size is normally adjusted by milling to provide a substantially uniform distribution in the formulation, e.g., about 40 mesh.

Some examples of suitable excipients include lactose, dextrose, sucrose, trehalose, sorbitol, mannitol, starches, gum acacia, calcium silicate, microcrystalline cellulose, polyvinylpyrrolidone, cellulose, water, syrup, and methyl cellulose. The formulations can additionally include lubricating agents such as talc, magnesium stearate and mineral oil, wetting agents, emulsifying and suspending agents, preserving agents such as methyl- and propylhydroxybenzoates, sweetening agents or flavoring agents. The compositions of the invention can be formulated so as to provide quick, sustained or delayed release of the active ingredient after administration to the patient by employing procedures well known in the art.

The compositions are preferably formulated in a unit dosage form with each dosage normally containing from about 50 μg to about 100 mg, more usually from about 1 mg to about 10 mg of the active ingredient. The term "unit dosage form" refers to physically discrete units suitable as unitary dosages for human subjects and other mammals, each unit containing a predetermined quantity of active material calculated to produce the desired therapeutic effect in association with a suitable pharmaceutical excipient.

For the purpose of parenteral administration, compositions containing a compound of the present invention preferably are combined with distilled water and the pH is adjusted to about 6.0 to about 9.0.

Additional pharmaceutical methods may be employed to control the duration of action. Controlled release preparations may be achieved by the use of polymers to complex or absorb a compound of the present invention. The controlled delivery may be exercised by selecting appropriate macromolecules (for example, polyesters, polyamino acids, polyvinylpyrrolidone, ethylenevinyl acetate, methylcellulose, carboxymethylcellulose, and protamine sulfate) and the concentration of macromolecules as well as the methods of incorporation in order to control release.

Another possible method to control the duration of action by controlled release preparations is to incorporate a compound of the present invention into particles of a polymeric material such as polyesters, polyamino acids, hydrogels, poly (lactic acid) or ethylene vinylacetate copolymers.

Alternatively, instead of incorporating a compound into these polymeric particles, it is possible to entrap a compound of the present invention in microcapsules prepared, for example, by coacervation techniques or by interfacial polymerization, for example, hydroxymethylcellulose or gelatin-microcapsules, respectively, or in colloidal drug delivery systems, for example, liposomes, albumin microspheres, microemulsions, nanoparticles, and nanocapsules, or in macroemulsions. Such teachings are disclosed in *Remington's Pharmaceutical Sciences* (1980).

The compounds of the present invention have insulinotropic activity. Thus, another aspect of the present invention provides a method for enhancing the expression of insulin comprising providing to a mammalian pancreatic B-type islet cell an effective amount of a compound of the present invention.

Similarly, the present invention provides a method for treating maturity onset diabetes mellitus in a mammal, preferably a human, in need of such treatment comprising administering an effective amount of a compound or composition of the present invention, to such a mammal.

The following examples are provided to further illustrate the present invention. It is not intended that the invention be limited in scope by reason of any of the following examples.

## Example 1

Individual aliquots of two GLP-1 molecules were lyophilized in small vials. Portions of 0.1M HEPES (N-[2-hydroxyethyl]piperazine-N'-[2-ethanesulfonic acid]) buffers at pH 7.4 containing various levels of zinc chloride were added to the aliquots to obtain a protein concentration of about 0.1 mg/mL. The samples were mixed and stored at ambient temperature (22° C) for about 18 hours. The mixtures were then centrifuged (Fisher Model 235C micro-centrifuge) for 5 minutes. The clear supernatants were pipetted from the tubes. The protein content of the supernatants was estimated by measuring their absorbance at 280 nm in a spectrophotometer (Gilford 260). The theoretical absorbance value for a 0.1 mg/mL solution of the GLP-1 molecules at this wavelength in the 1 cm cuvettes is 0.207. The results of this experiment are shown in Table 1.

**Table 1**

| Zn/GLP-1 Molecule | 280 nm Absorbance | |
|---|---|---|
| Molar Ratio | GLP-1 (7-36)amide | $Gly^8$-GLP-1 (7-36)amide |
| 0 | 0.172 | 0.136 |
| 0.3 | 0.099 | 0.079 |
| 0.5 | 0.057 | 0.070 |
| 0.7 | 0.035 | 0.058 |
| 1.0 | 0.039 | 0.057 |
| 3.0 | 0.048 | 0.044 |

This example shows that only a small quantity of zinc is required to complex with and precipitate a significant portion of the GLP-1 molecules from these dilute solutions.

## Example 2

5 mg of GLP-1 (7-36)amide was completely dissolved in 2.5 mL of pH 7.4, zinc-free 0.1M HEPES buffer. An additional 2.5 mL of pH 7.4, 0.1M HEPES buffer containing 0.6 mM zinc chloride was quickly added. The approximate molar ratio of zinc to GLP-1 (7-36)amide in this sample is 1 to 1. The solution immediately became cloudy and precipitation soon formed. The mixture was stored at ambient temperature (22° C) for 18 hours.

The precipitate became firmly attached to the bottom of the glass vial. The supernatant was completely decanted by pipette. The precipitate was then completely dissolved in 5.0 mL of 0.01N hydrochloric acid. The absorbance at 280 nm was determined for both the supernatant and redissolved precipitate solutions. The zinc levels in these solutions were quantitated by atomic absorption spectrophotometry. The results of this experiment are shown in Table 2.

**Table 2**

| | 280 nm Absorbance | Zinc Concentration in Parts per Million |
|---|---|---|
| Supernatant (5 ml) | 0.118 | 9.02 |
| Redissolved Precipitate (5 ml) | 1.932 | 13.3 |

This example shows that most of the GLP-1 (7-36)amide precipitated from the solution when the zinc-containing HEPES solution was added. The 280 nm absorbance value of 1.932 indicates the GLP-1 (7-36)amide concentration of the redissolved precipitate is 0.933 mg/ml, or 283 $\mu$M. The zinc concentration of this same solution, 13.3 parts per million, is equivalent to a zinc concentration of 203 $\mu$M. Therefore, the molar ratio of zinc to GLP-1 (7-36)amide in the precipitate was 0.717 to 1.

## Claims

1. A compound comprising a GLP-1 molecule having at least one histidine functionality or a modified histidine functionality and further having an isoelectric point in the range from about 6.0 to about 9.0, complexed with a divalent metal cation.

2. A compound of Claim 1 wherein said histidine functionality or modified histidine functionality is located at the amino-terminus (His[7]) of said GLP-1 molecule.

3. A compound as claimed in Claims 1 or 2 wherein said divalent metal cation is zinc.

4. A compound as claimed in any one of Claims 1 to 3 wherein said GLP-1 molecule is GLP-1 (7-36)amide.

5. A compound as claimed in any one of Claims 1 to 3 wherein said GLP-1 molecule is glycine-8-GLP-1 (7-36)amide.

6. A pharmaceutical formulation comprising as an active ingredient a GLP-1 compound, or a pharmaceutical acceptable salt thereof, as claimed in any one of Claims 1 to 5 associated with one or more pharmaceutically acceptable carriers therefor.

7. A GLP-1 compound, as claimed in any one of Claims 1 to 6, or a pharmaceutically acceptable salt thereof, for use in treating Type II diabetes (non-insulin dependant diabetes mellitus).

**European Patent Office**

## EUROPEAN SEARCH REPORT

Application Number

EP 94 30 8950

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | EP-A-0 619 322 (PFIZER) 12 October 1994<br>* page 7, line 4 - page 8, line 12; examples 21-32 *<br>* claims; figure 10; examples 34,37,38,46 * | 1-4,6,7 | C07K14/605<br>A61K47/48<br>A61K47/02 |
| P,X | EP-A-0 587 255 (GEN HOSPITAL CORP) 16 March 1994<br>* page 4, line 44 - line 47; claims; examples * | 1-3,6,7 | |
| X | WO-A-87 06941 (GEN HOSPITAL CORP) 19 November 1987<br>* page 9, last paragraph - page 10, paragraph 1; claims; examples * | 1-3,6,7 | |
| X | WO-A-90 11296 (GEN HOSPITAL CORP) 4 October 1990<br>* page 12, paragraph 3; claims; examples * | 1-3,6,7 | |
| D,A | WO-A-91 11457 (BUCKLEY DOUGLAS I ;HABENER JOEL F (US); MALLORY JOANNE B (US); MOJ) 8 August 1991<br>* the whole document * | | TECHNICAL FIELDS SEARCHED (Int.Cl.6)<br><br>C07K<br>A61K |

The present search report has been drawn up for all claims

| Place of search | Date of completion of the search | Examiner |
|---|---|---|
| THE HAGUE | 31 March 1995 | Fuhr, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)